# EUROPEAN PATENT APPLICATION

(11) **EP 1 038 972 A2**
(43) Date of publication of application: **27.09.2000**
(21) Application number: 00302295.1
(22) Date of filing: 21.03.2000
(51) Int. Cl.: C12Q 1/02

(54) **Method for investigating surfaces**

(30) Priority: 22.03.1999 US 273877
(71) Applicant: S.C. JOHNSON & SON, INC., Racine, WI 53403-2236 (US)
(72) Inventor: Avery, Richard W., Racine, WI 53402 (US); Rees, Gareth, Kingsley, Hampshire GU35 9JP (GB)
(74) Representative: Jones, Alan John

(57) **Abstract**

The tendency of a surface to accumulate biofilm is investigated by immersing a test piece having a surface to be investigated in a liquid culture of a microrganism which forms a biofilm. The culture is in a restricted growth medium and the surface is immersed so as to be freely drainable. After a pre-determined time the test piece is removed from the liquid culture and the concentration of microrganism bound to the test piece is determined, e.g. by removing the microrganisms from the surface and dispersing the microrganisms into a liquid culture.

## Description

### Technical Field

The present invention relates to a method for investigating the tendency of surfaces to accumulate biofilm.

### Background Art

When the surface of an object is exposed to a liquid medium capable of supporting the growth of microorganisms, a film of material comprising microorganisms, materials excreted by the microorganisms, and other materials present in the liquid medium which are trapped by the microorganisms or excreted products may form on the surface. This film will be referred to in this specification as biofilm. The formation of biofilm is usually undesirable. It may form stains on surfaces such as toilet bowls, which are unsightly and may trap harmful organisms. If it forms on the interior wall of a pipe it may restrict or block the flow of liquid through the pipe. Biofilm may form on surfaces which are permanently immersed in liquid and also on surfaces which are only intermittently in contact with liquid.

It is possible to reduce the tendency for biofilm to accumulate by suitable choice of the surface or of treatments applied to the surface. In order to compare the efficacy of various treatments it is necessary to find a method of comparing the tendency of various surfaces to accumulate biofilm.

One method of evaluating the tendency of a surface to accumulate biofilm is to immerse a specimen in a growth medium inoculated with a suitable microorganism and observing the quantity of biofilm which forms on the specimen in a given time. However, this requires the use of a medium which promotes the growth of the microorganism and this may lead to uncontrolled proliferation of the microorganism. It will then be more difficult to discriminate between the tendency of different surfaces to accumulate biofilm. If the rate of growth of the microorganism is restricted, then a relatively long time may be required to obtain observable levels of biofilm production.

There is a need for a method of evaluating the tendency of a surface to accumulate biofilm which provides improved discrimination between different surfaces and which can be carried out relatively quickly as a screening test to identify surface treatments for further study.

### Disclosure of Invention

According to the present invention a method of investigating the tendency of a surface to accumulate biofilm comprises the successive steps of:
(a) immersing a test piece having a surface to be investigated in a liquid culture of a microorganism which forms a biofilm, the culture being in a restricted growth culture medium, such that the surface to be investigated is freely drainable,
(b) allowing the test piece to remain in the liquid for a pre-determined period of time,
(c) removing the test piece from the liquid, and
(d) determining the concentration of microorganism bound to the test piece.

In an advantageous embodiment of the invention the concentration of microorganisms bound to the surface is determined by dispersing the microorganisms into a liquid culture medium, and determining the concentration of microorganisms in the liquid culture medium, as is discussed in more detail below.

In a further form of the invention the method comprises the step of determining the concentration of microorganism in the liquid culture from which the test piece has been removed.

Only one surface (hereinafter identified as the relevant surface) of the test piece may be of interest for the purpose of studying the tendency for biofilm accumulation. Thus, if the tendency for the accumulation of biofilm on glazed ceramic surfaces (e.g. toilet bowls) is of interest, then the test piece may be a portion of glazed ceramic tile, which will be glazed only on its front face. While it may be possible to dispose the test piece so that only the relevant surface is in contact with the liquid medium it will generally be more convenient to immerse the whole test piece in the liquid medium. Provided that only the surface under investigation differs between different test pieces the method will give useful information about the surface.

The test piece is so disposed in the liquid that the surface under investigation is freely drainable. A surface is "freely drainable" if liquid will drain from it if the liquid medium is withdrawn from around it. Thus, a substantially flat ceramic tile having one glazed surface and which is mounted vertically in the liquid will have a freely drainable glazed surface. If the same tile is mounted horizontally, the liquid will tend to be retained on the tile and it will not be freely drainable. The use of horizontally disposed tiles gives unsatisfactory results in this method.

The growth medium is one which keeps the microorganism alive so that it does not enter the "death phase", but does not allow the high rate of growth characteristic of microorganisms which have unrestricted access to nutrients. Suitable restricted growth media for specific types of microorganisms will be well known to the skilled person. The method involves bringing the microorganisms in the growth medium into contact with the surface to be investigated and thus requires a liquid medium (a broth) as opposed to a gel medium.

In general the microorganism forming a biofilm will be a bacterium or a mixture of bacteria. On surfaces exposed to light the formation of algal biofilms is possible and appropriate organisms can be used to investigate them, but the formation of bacterial biofilm will usually be of most interest in domestic, office, and most industrial situations. It will generally be more convenient for carrying out comparisons to use a pure culture of a bacterium rather than a mixture of different bacteria. Examples of bacteria which may be used are *Klebsiella* species, e.g. *Klebsiella pneumoniae.*

The liquid culture may conveniently be prepared by inoculating the growth medium and culturing it overnight. The concentration of microorganism in the medium when the test piece is introduced may conveniently be in the of from about 10⁵ to about 10⁷, perferably 10⁶ per cubic centimeter

The method of the present invention relies on obtaining information about a test piece from the liquid medium with which it has been in contact. It therefore follows that only one test piece can be immersed in a given pool of liquid medium (unless the test pieces are identical and more than one test piece is used to increase the surface area in contact with the medium). However, it will generally be desirable to use a single batch of liquid culture, to divide it into at least two substantially identical portions, and to immerse a test piece in each portion of liquid culture, and to maintain the portions of liquid culture and the test pieces under the same conditions until the concentration of the microorganism is determined. At least two identical test pieces may be used in order to check the reproducibility of the method and to provide an average result. However, it will generally be desirable to use test pieces which are identical except for the nature of a surface to be investigated.

Thus where the effect of surface treatments on biofilm formation on a given surface is being investigated it may be desirable to provide a plurality of test pieces, each of which has a surface subjected to a different treatment, and to test them simultaneously under the same conditions using the same liquid culture. As indicated above it will be desirable to provide a plurality of test pieces having a surface which has been subjected to a given treatment, so as to compensate for individual variations by obtaining an average result. In other words, the liquid culture is divided into inn portions, where m is the number of different surface treatments to be investigated and n is the number of test pieces which have been subjected to a given surface treatment. Preferably n is at least 10, more preferably at least 30, in order to give sufficient number of results to apply statistical methods.

In order to provide adequate contact between the culture and the test piece it is desirable to agitate the culture.

The test is usually most conveniently carried out at ambient temperature, e.g. 15°C to 30°C, preferably 20°C to 25°C. If the tendency of biofilm to form on surfaces at higher or lower temperature is being investigated then it may be desirable to use a microorganism which has a preference for that temperature and to adjust the temperature of the test accordingly. However, as the method is directed to indicating the tendency of the microorganism to attach to the surface rather than to indicate its rate of growth on the surface it may be possible to obtain useful results in tests carried out without special cooling or heating arrangements.

The time for which the liquid culture is allowed to remain in contact with the test piece may vary over a moderately wide range, e.g. 12 to 36 hours, e.g 18 hours

The concentration of microorganisms bound to the surface of the test piece is most conveniently determined by dispersing the microorganisms bound to the surface into a liquid culture medium, and then determining the concentration of microorganism in the liquid medium. The use of a restricted growth culture medium in the initial stage of the test gives an important advantage in the determination of the concentration of the microorganisms bound to the surface. Using a culture medium formulated to maximize growth of the microorganism would tend to give a high level of microorganism on the surface which when dispersed into a liquid medium would give an excessively high concentration of microorganisms. It would be more difficult to investigate and evaluate such a liquid.

The number of microorganisms attached to the surface of the test piece may be determined by washing away any residual culture medium, immersing the test piece in a new liquid culture medium, mechanically dislodging any microorganisms attached to the relevant surface into the culture medium, and determining the concentration of microorganisms in the new culture medium. The concentration of microorganisms in the new liquid culture may be determined by conventional methods. Thus, samples of the culture, after dilution to a known extent, may be dispersed over an agar nutrient medium. The number of colonies obtained after culturing for an appropriate time are then determined. The skilled person will understand that the liquid medium into which the microorganisms attached to the surface are dispersed prior to dilution and application to an agar nutrient medium need not itself be a nutrient medium provided that the viability of the microorganisms is not affected.

The determination of the concentration of microorganisms in the liquid culture medium from which the test piece is removed gives valuable additional information about the affinity for microorganisms of the surface under investigation. A low concentration of microorganisms in the liquid culture medium indicates that the surface has a high affinity for microorganisms. The concentration may be determined by conventional dilution methods as indicated above.

The test method of the present invention gives results for the concentrations of microorganisms. Processes involving the growth of microorganisms tend to give inherently variable results, and for this reason it will be generally desirable to carry out multiple tests to obtain average results. It is possible to calculate arithmetic means for the results of multiple tests. It may be preferred to calculate geometric means as the geometric mean reduces the effect of a few excessively high or low results. If sufficient multiple test results are available it may be possible to apply statistical methods such as variation about the mean or T-test to check whether any differences found are significant.

Persons skilled in microbiological measurements will be familiar with the mathematical methods used to evaluate such test data and will be able to select the appropriate techniques to apply to results produced by the method of the invention.

### Best Mode of Carrying Out the Invention

### Example 1

Ceramic tiles, from a single batch which were glazed on one surface, were cut into sections, each 2.5 cm x 2.5 cm. Each tile section had all four sides cut to ensure that the absorption of liquid by the unglazed surfaces of each section was as consistent as possible.

Ten sections were treated with an appropriate volume of a liquid containing an agent whose effect on biofilm accumulation was to be tested. The sections were then subjected to an identical rinsing regime to remove all traces of the agent which were not bound to the tile. The sections were all labelled A-M to facilitate identification.

Ten sections from the same batch were used as controls and were not treated with any agent.

The tile sections were introduced into pre-sterilized ice trays. These were trays normally used for the production of ice cubes and contained twelve non-communicating compartments or wells capable of retaining liquid. Each tile section lodged vertically within a well without the need for any additional fixing device. Each well contained 6.5cm³ of a broth culture of *Klebsiella pneumoniae* which had been prepared the previous day and allowed to stand overnight. The culture medium was a restricted growth culture medium, namely 1% peptone.

The *Klebsiella pneumoniae* culture concentration was adjusted to a target inoculum of approximately 10⁶ per cm³.

The inoculated ice trays, loaded with treated or control tiles, were mounted on to a flat bed rotary shaker. A total of six such trays could be processed at a time, each tray embedded into an agar base to locate it securely.

The inoculated trays were rotated for 18 hours at ambient temperature (about 20°C) on the flat bed rotatory shaker at a constant rate to maintain a consistent flow cycle within each well.

After the tiles had been exposed to the broth culture for 18 hours, 1 cm³ of broth was removed from each well, serially diluted in Ringer's solution (a well-known physiological medium), pour plated, and the number of viable bacteria estimated. This procedure was repeated for a total of 10 inoculated wells and a mean calculated for each series of 10 tile sections subjected to the same initial treatment. This procedure determines the concentration of freely dispersed (planktonic) bacteria.

The concentration of microorganisms attached to the surface was also estimated. The individual tile sections were removed and rinsed with sterile distilled water for 30 seconds.

After rinsing, the tile sections were transferred to 10 cm³ of sterile Ringer's solution and the front (glazed) face of the tile section abraded rigorously with polytetrafluoroethylene scraper for a total of 15 seconds. The resulting inoculum was then thoroughly homogenized. After homogenization, 1 cm³ of the inoculum was removed, serially diluted, and pour plated to estimate the number of viable bacteria that remained attached to or settled on to the vertical face of the tile section.

The results are summarized in Table 1. Both arithmetic means and geometric means we given. The arithmetic mean is the sum of all the bacterial concentrations for each group of 10 tile sections divided by 10. The geometric mean is the 10th root of the product of the bacterial concentrations for each group of 10 tile sections.

**TABLE 1**

| Planktonic | | | Biofilm | |
|---|---|---|---|---|
| | | | | |

| | Arithmetic mean | Geometric mean | Arithmetic mean | Geometric mean |
|---|---|---|---|---|
| A | 580 | 2.28 | 10565 | 3.75 |
| B | 14871 | 3.67 | 44608 | 4.64 |
| C | 17300 | 3.70 | 36060 | 4.34 |
| D | 315 | 1.85 | 18660 | 4.04 |
| E | 5930 | 3.26 | 12320 | 3.85 |
| F | 130 | 2.06 | 694 | 2.59 |
| G | 27320 | 4.31 | 85960 | 4.84 |
| H | 9610 | 3.44 | 31 | 1.21 |
| J | 690 | 2.44 | 660 | 2.26 |
| K | 2030 | 2.92 | 177 | 1.84 |
| L | 2140 | 2.85 | 99 | 1.99 |
| M | 2740 | 2..91 | 170 | 2.17 |

## Claims

1. A method for investigating the tendency of a surface to accumulate biofilm, comprising the successive steps of
(a) immersing a test piece having a surface to be investigated in a liquid culture of a microorganism which forms a biofilm, the culture being in a restricted growth culture medium, said test piece positioned in a manner such that the surface to be investigated is freely drainable,
(b) allowing the test piece to remain in the liquid for a pre-determined period of time,
(c) removing the test piece from the liquid, and
(d) determining the concentration of microorganism bound to said surface to be investigated of the test piece.

2. A method according to Claim 1 wherein the concentration of microorganisms bound to the surface is determined by dispersing the microorganisms into a liquid culture medium, and determining the concentration of microorganisms in the liquid culture medium.

3. A method according to Claim 2 which comprises the additional step of determining the concentration of microorganism in the liquid culture from which the test piece has been removed.

4. A method according to Claim 1 wherein the test piece is a glazed ceramic tile.

5. A method according to Claim 1 wherein the liquid culture is a culture of a bacterium.

6. A method according to Claim 5 wherein the culture is a culture of a *Klebsiella* species.

7. A method according to Claim 6 wherein the liquid culture contains 10⁵ to 10⁶ per cubic centimeter microorganisms when it is brought into contact with the test piece.

8. A method according to Claim 7 wherein a single batch of liquid culture is divided into at least two substantially identical portions and a test piece is immersed in each portion, and the portions of liquid culture and the test pieces are maintained under the same conditions until the concentrations of microorganisms are determined.

9. A method according to Claim 6 wherein there are a plurality of test pieces, each of which has a surface subjected to a different treatment, and the liquid culture is divided into m times n portions, where m is the number of different surface treatments being investigated and n is the number of test pieces which have been subjected to any given surface treatment.

10. A method according to Claim 9 wherein n is at least 10.

11. A method according to Claim 10 wherein the liquid culture in contact with the test piece is agitated.
